(19) Europäisches Patentamt
European Patent Office
Office européen des brevets

(11) **EP 3 752 131 B1**

(12) **EUROPEAN PATENT SPECIFICATION**

(45) Date of publication and mention of the grant of the patent:
**02.06.2021 Bulletin 2021/22**

(21) Application number: **19710504.2**

(22) Date of filing: **12.02.2019**

(51) Int Cl.:
*A61K 9/127* (2006.01)    *A61K 47/02* (2006.01)
*A61K 47/12* (2006.01)    *A61K 47/36* (2006.01)
*B01F 13/00* (2006.01)

(86) International application number:
**PCT/IT2019/050028**

(87) International publication number:
**WO 2019/159210 (22.08.2019 Gazette 2019/34)**

(54) **CONTINUOUS PROCESS FOR COATING LIPOSOMIAL VECTORS WITH POLYMER**

KONTINUIERLICHES VERFAHREN ZUR BESCHICHTUNG VON LIPOSOMALEN VEKTOREN MIT POLYMER

PROCÉDÉ CONTINU DE REVÊTEMENT DE VECTEURS LIPOSOMIAUX PAR UN POLYMÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2018 IT 201800002631**

(43) Date of publication of application:
**23.12.2020 Bulletin 2020/52**

(73) Proprietor: **ENG4LIFE S.R.L.**
**83100 Avellino (IT)**

(72) Inventors:
• **BARBA, Anna Angela**
**83100 Avellino (FR) (IT)**
• **LAMBERTI, Gaetano**
**83100 Avellino (FR) (IT)**
• **D'AMORE, Matteo**
**83100 Avellino (FR) (IT)**
• **BOCHICCHIO, Sabrina**
**83100 Avellino (FR) (IT)**
• **DALMORO, Annalisa**
**83100 Avellino (FR) (IT)**

(74) Representative: **Banchetti, Marina et al**
**Barzanò & Zanardo Roma S.p.A.**
**Via Piemonte 26**
**00187 Roma (IT)**

(56) References cited:
• **S.H. BANG ET AL: "Influence of chitosan coating on the liposomal surface on physicochemical properties and the release profile of nanocarrier systems", JOURNAL OF MICROENCAPSULATION., vol. 28, no. 7, 23 August 2011 (2011-08-23), pages 595-604, XP055510064, GB ISSN: 0265-2048, DOI: 10.3109/02652048.2011.557748**
• **Sabrina Bochicchio ET AL: "Phenomenological and Formulation Aspects in Tailored Nanoliposome Production" In: "Liposomes", 25 October 2017 (2017-10-25), InTech, XP055451295, ISBN: 978-953-51-3580-7 DOI: 10.5772/intechopen.68157, point 2.2 on pages 35-36, fig. 3**
• **XU J H ET AL: "Preparation of monodispersed chitosan microspheres and in situ encapsulation of BSA in a co-axial microfluidic device", BIOMEDICAL MICRODEVICES, KLUWER ACADEMIC PUBLISHERS, BO, vol. 11, no. 1, 23 September 2008 (2008-09-23), pages 243-249, XP019670965, ISSN: 1572-8781**

## Description

## Field of the invention

The present invention concerns a process for coating liposomial nano-vectors with polymer by means of a continuous process, as well as the coated nanostructures thus obtained. More particularly, the invention relates to a continuous, and therefore rapid and highly productive, process for obtaining nanoliposomes completely coated with chitosan, a biocompatible natural polymer able to increase their stability and mucoadhesivity. Such process uses a highly productive "simil-microfluidic" technique for the polymeric coating of nano-vectors.

## Background of the invention

As is known, liposomes are vesicles consisting of one or more double layers of phospholipids containing an aqueous core; being very similar to biological membranes, liposomes are important systems for the administration of drugs and other biologically active molecules. In recent years, liposomes have found wide use in various fields of application, such as pharmaceutical, nutraceutical and cosmetic fields.

In fact, the liposomal carriers are structures which can carry active ingredients through different administration routes, first of all through the oral and the injection route. Currently, liposomes are among the most used carriers, because they offer great advantages both in terms of biocompatibility and in terms of preparative versatility. Their biocompatibility is ensured by their own composition and structure, which mimics the cell membrane and therefore makes them easily absorbed by the cells, while from a preparatory point of view, they offer the advantage of having their size set on micro- or nanoscale depending on the procedure adopted for their production. Moreover, they have the additional advantage of being capable to be functionalized.

Although liposomes are characterized by a high level of biocompatibility, biodegradability and low toxicity, their poor stability in biological fluids and in storage conditions, as well as their susceptibility to being easily degraded in the gastrointestinal tract by pH, bile salts and pancreatic lipases, have led to the development of new approaches for their stabilization.

The modification of nanoliposomes' surface by means of natural polymers is an already used and very promising strategy to counteract their high tendency to be degraded in extra-cellular physiological environments, and their tendency to aggregate.

Among the different polymers that are used to promote liposomal stabilization, such as polyethylene glycol (PEG) or acrylic copolymers, chitosan is preferred for its high affinity with biological membranes. Chitosan is a polysaccharide composed of randomly distributed units of D-glucosamine and N-acetyl-D-glucosamine bound by $\beta$(1-4) bonds, which is obtained by partial deacetylation of the amine groups of a natural polysaccharide, chitin. Chitin is the second most abundant biopolymer in nature after cellulose, and derives mostly from the exoskeleton of insects and crustaceans. Chitosan is a hydrophilic, cationic, biocompatible and biodegradable polymer that has low toxicity, high biocompatibility, absorptive capacity and is non-antigenic.

It has been shown that the cationic nature of chitosan at pH 6.5 - 7 is responsible for the strong attraction for mucin (property known as mucoadhesion), a negatively charged glycosylated protein. This protein is highly concentrated on the surface of some tissues such as pulmonary, corneal, intestinal and gastric tissues (Karn, P.R. et al., Mucoadhesive liposomal delivery systems: the choice of coating material, Drug development and industrial pharmacy, 2011, 37(4), 482-488). Thanks to its mucoadhesion characteristics, chitosan is used in controlled delivery formulations for ophthalmic, nasal and oral use, and is receiving increasing attention in the design of new bioadhesive systems for the delivery of therapeutic agents. A coating with chitosan allows an increase in residence time at the site of action of carriers containing an active ingredient, and at the same time increases the paracellular permeability through the interaction between the positive charges of the polymer and the negative charges which characterize the enterocytes (thus promoting the absorption of the active molecule) (Wu, Z.-H. et al., Hypoglycemic efficacy of chitosan-coated insulin liposomes after oral administration in mice, Acta Pharmacologica Sinica, 2004, 25(7), 966-972).

For all these favorable characteristics, chitosan is therefore used as a stabilizing agent for liposomes. In fact, by combining the characteristics of nanoliposomes with those of chitosan it is possible to obtain stable coated nanostructures, with mucoadhesive properties, suitable for a controlled and effective delivery of the active molecules encapsulated.

There are several techniques that have been used so far for the surface coating of nanoliposomes with polymer. Among them, the most applied or studied ones at the research level are the following:

- coating by simple contact, incubation and mixing of chitosan solutions and liposomal suspensions;
- coating by the dripping method (*drop-wise*);
- coating by sonication and evaporation of the solvent (*reverse-phase evaporation,* REV).
- coating by inverse supercritical evaporation (*Improved Supercritical Reverse-Phase Evaporation,* ISCRPE);
- coating by electrostatic deposition (*Electrostatic Deposition,* ED);
- coating by layering (*Layer-by-Layer deposition,* LdL).

The coating of preformed liposomes by steps of contact, incubation and mixing with chitosan, which is the most used technique, involves the addition of a chitosan solution to an equal volume of a suspension containing previously homogenized lipid vesicles, followed by an incubation at 10 °C for about 1 hour (Wu, Z.-H. et al., cited above). This method has been used, for example, in the patent US 8658204 (Otsuka Pharmaceutical Co., Ltd., *Transpulmonary liposome for controlling drug arrival*) for the production of liposomes coated with polyvinyl alcohol or with chitosan for an intrapulmonary drug delivery.

The same technique had been described in the international patent application publ. No. WO 2006/062506 (Enzrel, Inc., *Chitosan-coated liposome drug delivery of antioxidant or anti-inflammatory compounds*). According to Example 3 of this document, a chitosan solution is mixed with proliposomes (dry and flowing granular structures which, upon addition of water, are dispersed to form multilaminar liposome suspensions) produced by a procedure proposed in the document itself. Subsequently, using a small syringe, a mixture of sodium hydroxide and ethyl alcohol is added, thus obtaining a gel which is incubated for 30 minutes. The proliposomes coated with chitosan are separated, washed in distilled water under neutral conditions and finally dried for at least 24 hours at room temperature.

The incubation of liposomes with chitosan is also used in the patent application US 2013/0337047 A1 (Zarzatech, Inc., *Compositions for treating oral and periodontal infections*) to cover cimetosomes (cimetidine-containing liposomes) produced according to the disclosure with low molecular weight chitosan.

According to the above-mentioned second technique for coating preformed polymeric liposomes, called drop-wise technique, chitosan is dissolved in water and dripped into an equal volume of a previously prepared liposomal suspension. During the drop-wise addition of the polymer solution, which may last several hours even for small batches, the liposomal suspension is placed under adequate stirring at the temperature of 20 °C (Guo, J.-x. et al., Chitosan-coated liposomes: characterization and interaction with leuprolide, International Journal of pharmaceutics, 2003, 260(2), 167-173). This technique has also been used in the patent application US 2014/0017298 A1 (The Administrators of the Tulane Educational Fund, *Biopolymer hooks to create coatings on liposomes*) for the production of liposomes coated with hydrophobically modified chitosan and in the article by Bang et al., Journal of Microencapsulation, 28:7, 595-604.

The reverse-phase evaporation (REV) technique for the production of chitosan-coated liposomes involves the formation of a water-in-oil emulsion (or in organic phase) starting from phospholipids solubilized in organic solvents, to which a solution of chitosan dissolved in acetate buffer is added. The mixture is sonicated, thus producing a homogeneous inverse micellar suspension, which is evaporated under reduced pressure at temperatures between 25 and 35 °C, in order to remove the organic solvent. After the addition of an aqueous phase, the formation of coated nano-vesicles occurs, and the tatter are finally subjected to homogenization for 10 minutes. (Gonçalves, M. C. et al., Chitosan coated liposomes as an innovative nanocarrier for drugs, Journal of biomedical nanotechnology, 2012, 8(2), 240-250).

The technique described above has been revisited in "Improved Supercritical Reverse-Phase Evaporation", in order to avoid the use of organic solvents, improve the stability of the vectors and increase the efficiency of encapsulation. (Otake, K. et al., One-step preparation of chitosan-coated cationic liposomes by an improved supercritical reverse-phase evaporation method, Langmuir, 2006, 22(9), 4054-4059). This technique provides for the formation of a mixture of phospholipids in aqueous phase, which is pressurized with $CO_2$ at the conditions of 200 bar and 60 °C, obtaining a $CO_2$/water emulsion. After 40 minutes the system is depressurized to generate liposomes. Chitosan can be added to the liposomal formula to produce lipid structures coated with chitosan (Meure, L. A. et al., Conventional and dense gas techniques for the production of liposomes: a review. Aaps Pharmscitech, 2008, 9(3), 798-809).

The production of polymer-coated liposomal structures by electrostatic deposition (Electrostatic Deposition) occurs by mixing previously prepared liposome suspensions with a polysaccharide solution having opposite charge. Negatively charged liposomal vesicles are added to a solution containing chitosan, i.e. a positively charged polymer, and the suspension is kept under constant stirring (480 rpm) for 10 minutes (Pistone, S. et al., Polysaccharide-coated liposomal formulations for dental targeting, International Journal of Pharmaceutics, 2017, 516(1), 106-115).

The principle of electrostatic deposition is also used in the Layer-by-Layer Deposition technique, by which liposomes coated by several polymeric layers are obtained. According to this technique, aliquots of sample containing positively charged vesicles, previously prepared, are first incubated with an alginate solution, i.e. a negatively charged polymer, and then left for one night in a rotating incubator. By ultracentrifugation, the polymer not adsorbed on the surface of the vesicles is removed, and the liposomes coated with alginate (negatively charged) are resuspended in a solution of chitosan (cationic polymer) and incubated again for one night. Finally, the non-adsorbed polyelectrolytes are removed by ultrafiltration by means of a mini-extruder with membranes having a pore size of 80 nm (Ge, L. e Ji, J., Fabrication and stability study of multilayer liposome, Asian Journal of Chemistry, 2010 22(1), 66). e & Ji, 2010).

The techniques described above, developed on a laboratory scale and used to date for the production of coated lipid structures, operate discontinuously on small volumes, and are very expensive in terms of energy and processing times, as they require different phases of process and often long operational times. In some of these methods it is necessary to operate under drastic conditions (high pressures), or to have recourse to a considerable use of organic solvents, which could remain in traces in the final product.

It should also be noted that, by means of the discontinuous techniques mentioned above, it is not always possible to obtain control over the coating of the lipid structures, which very often are characterized by an uneven polymeric surface. A chitosan layer distributed in a non-homogeneous manner on the surface of the vesicles implies a greater propensity to aggregate between the particles, with the consequent loss of stability. Moreover, any non-coated surface portion of the liposomes allows the establishment of degradation phenomena on the encapsulated active principles by the action of biological fluids, or their early release into the storage fluids.

The drastic conditions sometimes required by said technologies, the small volumes with which they operate, the process discontinuity and the lack of control over the final characteristics of the vectors produced reveal the inappropriateness of the use of such methods on an industrial scale. Even the most recent and relatively new techniques, such as supercritical reverse phase evaporation (Improved Supercritical Reverse-Phase Evaporation, ISCRPE) exploit expensive apparatuses and are characterized by relatively low productivity.

It should also be noted that, in the last decade, scientific research has focused on refining strategies to create coated complexes uniquely from a formulative point of view, while no new solution has been proposed for the optimization of production processes.

In the light of the foregoing, the present invention is aimed at providing a technique for producing nanoliposomal vectors perfectly coated with chitosan, having mucoadhesive and stable properties, which is cheaper and faster than those described so far, and allows to obtain high yields through a rapid and continuous process, with high productivity.

## Summary of the invention

The polymer surface coating technique of nanoliposomes proposed according to the invention originates from a previous invention of the same current research group, described in the unpublished prior Italian patent application No. 102017000099627, filed on 6 September 2017. This application concerns a process for the continuous production of nanoliposomial vectors which uses a new high-performance "simil-microfluidic" technique for the formation of nanoliposomes, coupled to an ultrasonic treatment for the homogenization of the obtained nanoliposomes.

The microfluidic device described in the previous patent application uses two volumetric pumps to respectively feed a lipid stream dissolved in an organic solvent and an aqueous stream containing an active ingredient to be encapsulated at a point of contact between the two streams. The latter, according to an exemplary embodiment, is made by means of a 0.6 mm internal diameter needle (lipid stream) inserted into a silicone tube with an internal diameter of 3 mm and a length of 185 mm, which constitutes the extension of the aqueous stream tube, wherein the formation of the nanoliposomes occurs. The described process therefore essentially results in a contact between two flows: on one side phospholipids, preferably consisting of phosphatidylcholine and cholesterol, in alcoholic solution, and on the other side an aqueous solution containing the active ingredient to be carried, possibly together with other useful water-soluble components. Such contact takes place inside a tubular device in which the interdiffusion phenomena between the two phases cause the formation of lipid vesicles of nanometric dimensions, containing inside them the active ingredient to be carried.

The cited ingredients are processed in the production method described in a continuous or semi-continuous manner, to obtain nanoliposomes, with an average size of less than, or equal to, 100 nm, and characterized by a low polydispersity index. The proposed production method, which derives from the microfluidic approaches but does not use micrometric size equipment, consists, in practice, of injecting an alcoholic solution of membrane lipids into a channel in which an aqueous solution containing an active ingredient flows. The flow rates and sizes of the pipes are selected in such a way that the fluids always flow at laminar flow regime. As already noted, the nanoliposomes are formed at the point of contact between the two solutions. Then, the resulting hydroalcoholic solution carrying the nanoliposomes in suspension is directed to a section where it undergoes an ultrasonic homogenization, which reduces the average size of the nanoliposomes, and makes them uniform, thus reducing their polydispersity.

The nanoliposomes of the prior patent application, therefore, are obtained by the following operations:

    a) producing a first alcoholic solution of phospholipids and cholesterol at a concentration of from 0,1 to 10 mg/mL;

    b) producing of a second alcoholic solution containing the active ingredient to be incapsulated, along with any other useful water-soluble ingredients;

    c) pumping said alcoholic and aqueous solutions through two volumetric pumps in flow rates of the order of mL/s and in a respective volume flow rate ratio of from 5:1 to 50:1, preferably 10:1;

    d) contacting the flows consisting of said two solutions by making them pass through a device operating in simil-microfluidic regime, which results in the formation of nanoliposomes by injection of said first solution stream into said second solution stream through a duct, for instance a hollow needle, of internal diameter below 1 mm, the duct of said second solution having an internal diameter of 2-5 mm and a length after the injection point of 150-200 mm;

    e) subjecting to an ultrasound treatment the nanoliposomes suspension obtained from the previous operation, to reduce the size of the nanoliposomes produced and limit their polydispersity, the nanoliposomes thus obtained having an average size not higher than 100 nm and a low polydispersity index.

Starting from the described nanoliposome production technique, according to the present invention a new method of polymer coating of liposomes has been devised that uses a similar microfluidic device for the mass production of lipid-polymer particles, consisting of liposomes uniformly coated with chitosan.

The technique proposed according to the invention is based on the contact between two streams: a suspension of nanoliposomes, previously produced, and a chitosan solution, respectively fed by two volumetric pumps. The liposomal stream, introduced for example by means of a 0.6 mm internal diameter needle, comes into contact with the polymer stream (chitosan in the aqueous phase) which flows through a tube, for example of silicone, with an inner diameter of 3 mm and 185 mm in length. It has been found that under these conditions the meeting between the two phases, one consisting of nanoliposomes in aqueous suspension and the other consisting of an acidic aqueous solution containing chitosan, produces uniformly polymer-coated nanometer-sized structures.

The process according to the invention is governed by the continuous and uniform electrostatic interaction between the negatively charged nanoliposomes and chitosan, cationic polyelectrolyte, which is established when the two phases begin to inter-diffuse, causing a reversal of the net surface charge of the lipid particles.

It is known from literature that the formation of liposomes coated with chitosan, which are stable and have no tendency either to agglomeration or to sedimentation, occurs at a concentration of chitosan comprised between two limits, referred to as minimum critical concentration ($c_{min}$) and maximum critical concentration ($c_{max}$). Below the $c_{min}$, that is, at too low polymer concentrations, the liposomes undergo a complete breakdown and rapidly sediment on the bottom of the container. This can be ascribed to the fact that the chitosan molecules may have been bound to the anionic phospholipid molecules by forming coacervates, rather than realizing a coating thereon. Instead, above the $c_{max}$, the excess of polymer creates a gradient of osmotic pressure due to the exclusion of polymer molecules from the immediate proximity of the surfaces of the particles, which then generates an attractive force which increases as the polymer concentration increases. At sufficiently high concentrations of polymer not adsorbed on the liposomes wall, aggregation of the particles occurs. In this context, the minimum polymer concentration (chitosan) required to cover particles of opposite charge (anionic liposomes) is called saturation concentration ($c_{sat}$), where obviously $c_{min} < c_{sat} < c_{max}$.

The concentration of chitosan required to saturate the surfaces of the $c_{sat}$ liposomes is directly proportional to the concentration of the liposomes and to the surface charge of the same (measurable by the zeta potential) and is inversely proportional to the average size of the particles. As a consequence, the formation of stable nanostructures depends on the structure of the encapsulated molecule in the liposomes, which can vary both the final size of the liposomes and the zeta potential. However, since the uncoated nanoliposomes produced according to the previous patent application have an average size of less than or equal to 100 nm and a surface charge of between -50 mV and -10 mV, according to the present invention the formation of stable nanostructures is achieved using chitosan at a concentration equal to or less than 0.01% w/v.

The lipid vesicles, which constitute the part subjected to coating, are preferably produced through the production process of nanoliposomal vectors of the aforementioned previous Italian patent application, but it is not excluded that liposomes of other origin can be used as starting material for the coating with chitosan according to the process of this invention.

According to the invention, lipid vesicles obtained by any process are made to flow inside the microfluidic device at the same volume flow used for the polymeric phase. The flow rates and sizes of the selected tubes play a key role in the production of stable and perfectly polymer-coated liposomal systems, and ensure that the fluids always flow under the laminar motion regime. These fluid-dynamics conditions allow a continuous and uniform interaction between equal volumes of liposomes and chitosan, overcoming the limitations of discontinuous techniques such as the drop-wise method in which a drop of chitosan is added to the entire volume of the liposomal suspension, with the likelihood that thus drop comes into contact with already coated liposomes, thus leading to the formation of coacervates.

By means of the proposed invention, using the cited materials and the indicated operating conditions, nanostructures coated with average size lower than 300 nm (value that derives from a numerical distribution) are obtained, having a low polydispersity index.

**Detailed description of the invention**

Therefore, the present invention specifically provides nanoliposomes or lipid nanostructures coated with chitosan for the administration of active molecules for pharmaceutical, nutraceutical or cosmetic use, obtainable by means of a process comprising the following operations:

a) providing, as starting material, a suspension of nanoliposomes optionally encapsulating one or more active ingredients to be administered, said nanoliposomes being composed of phospholipids, preferably phosphatidylcholine, and cholesterol, preferably used in molar ratio 2.5:1, at a total lipid concentration between 0.1 and 10 mg/mL, preferably equal to 5 mg/mL;

b) producing an aqueous solution preferably containing between 0.2% and 1.0% by volume of acetic acid and containing chitosan at a concentration of between 0.005% and 0.015% w/v, preferably not higher than 0, 01% w/v;

c) pumping of said suspension of nanoliposomes and aqueous solution containing chitosan through two volumetric pumps in a volumetric flow ratio of between 0.8:1 and 1.2:1 (preferably at the same volumetric flow rate of 25 mL/min);

d) contacting the flows consisting of said suspension of nanoliposomes and said chitosan-containing solution by making them pass through a simil-microfluidic device, which effects an inter-diffusion of said nanoliposomes suspension into the stream of said aqueous solution by passage through a duct, for instance a hollow needle, of internal diameter below 1 mm, the duct of said asolution having an internal diameter of 2-5 mm and a length after the insertion point of 150-200 mm, thereby obtaining nanostructures coated with chitosan;

e) collecting the suspension of the coated nanostructures obtained from the previous production step.

Further characteristics of the chitosan-coated nanoliposomes according to the invention and of the process for their production are set forth in the further claims.

The liposomal suspension constituting the starting material must contain nanoliposomes produced starting from phospholipids such as lecithin (phosphatidylcholine, PC), and membrane lipids such as cholesterol (COL), preferably in a molar ratio PC:COL = 2.5:1, which ratio replicates the cell membrane composition, and thus promotes fusogenic actions and transfection.

According to preferred embodiments of the invention, as already noted, the total lipid concentration of the starting nanoliposomes is 5 mg/mL. Such nanoliposomes may have been obtained by means of a process for producing nanoliposomal vectors which makes use of the simil-microfluidic technique described in the prior unpublished Italian patent application No. 102017000099627, or may have been produced by different techniques.

The starting nanoliposomes, which are intended to be coated with chitosan, may contain any active ingredient or functional molecule, such as active pharmaceutical ingredients, vitamins, food supplements, colorants. Some examples of molecules to which the coating process of the invention has been applied include indomethacin (CAS n.53-86-1), vitamin $K_2$ (CAS n. 863-61-6), alpha-tocopherol (CAS n. 10191-41-0), vitamin $K_3$ (CAS n. 58-27-5), vitamin $D_3$ (CAS n. 67-97-0) and curcumin (CAS n. 458-37-7).

Other ingredients, which are interesting for a coating with chitosan with the method according to the invention, in particular with a view to preparing mucoadhesive delivery systems for sublingual administration, are, for example, melatonin, vitamin B12, resveratrol, policosanol, glutathione and boswellic acids. In general, however, the coated liposomes can be used for the delivery of antibiotics (of which indomethacin is an example), antineoplastic drugs (such as doxorubicin: Deygen, I. M. et al., Novel Prodrug of Doxorubicin Modified by Stearoylspermine Encapsulated into PEG-Chitosan-Stabilized Liposomes. Langmuir 2016, 32, 10861-10869), peptides (see, e.g., Werle, M. et al., Chitosan-aprotinin coated liposomes for oral peptide delivery: development, characterization and in vivo evaluation. International Journal of Pharmaceutics 2009, 370 (1-2), 26-32) and vaccines (Channarong, S. et al., Development and evaluation of chitosan-coated liposomes for oral DNA vaccine: the improvement of Peyer's patch targeting using a polyplex-loaded liposomes. AAPS Pharmscitech, 2011, 12(1), 192-200).

It is known that chitosan is poorly water-soluble at neutral pH, but is soluble in acidified aqueous solutions. For this reason, according to the preferred embodiments of the invention, the aqueous solution containing chitosan contains between 0.2% and 1.0% by volume of an organic or inorganic acid, preferably an acid organic selected from acetic acid, adipic acid, formic acid, lactic acid, malic acid, propionic acid and succinic acid. In a particularly preferred manner, the aqueous chitosan solution contains 0.5% by volume of acetic acid.

Preferably, the chitosan used has an average molecular weight, and 75%-85% of deacetylation. The viscosity of a 1% solution of chitosan in 1% acetic acid is 200-800 cP (supplier Sigma Aldrich s.r.l. Milano).

As already noted, the polymeric solution must be prepared using preferably chitosan at a concentration equal to or less than 0.01% w/v. In this case it is possible to use the reference of 10 mg of chitosan dissolved in 10 ml of an aqueous solution of acetic acid at 0.5% (v/v), stirred for 90 minutes at room temperature, and then diluted until obtaining the concentration of 0.01% w/v.

Chitosan concentration is a process parameter correlated to the volumetric fraction of the nanoliposomes to be coated (approximate to the mass fraction of the lipids that make up the vesicles), to the polyelectrolyte load on the surface of the nanoliposomes and to the medium radius of the vesicles to be coated. As already noted, a polymer concentration higher than that used in the present invention causes aggregation and flocculation phenomena of the obtained structures.

In more detail, the process for producing nanostructures coated according to the invention may be described as comprising the following unitary operations:

A. Providing an uncoated liposomal suspension as starting material, and storing it in a first vessel connected to the next pumping section;

B. Preparing the aqueous solution containing chitosan and storing itf in a second stirred vessel connected to the next pumping section;

C. Pumping the two solutions using peristaltic pumps or other volumetric devices. Peristaltic pumps are preferred in view of the negligible interaction with pumped fluids and for their easy process scalability;

D. Injecting the liposomal suspension by means of a needle into the tube where the aqueous polymeric solution flows. The parameters of selected processes, such as the internal diameters of the pipes and the flow rates, ensure that the flows are in laminar regime. Furthermore, the length of the piping of the polymeric solution is such as to exclude inlet effects on the development of the velocity profile. The fluid-dynamic conditions replicate those observed in the micro-fluidics field. In the proposed process, however, the systems have dimensions on a millimeter scale, avoiding the difficulties and costs of implementation, and the energy commitment typical of microfluidic systems. As a reference value, an inside diameter of the needle of 0.6 mm and an inner diameter of the tubing for the aqueous solution of 3.0 mm can be assumed. This choice corresponds to a ratio of two diameters equal to 1:5, which is therefore a reference value for this parameter. The flow rate of the polymer solution and the flow rate of the liposomal suspension should preferably be equal, and preferably of 25 mL / min;

E. Collecting the suspension of lipid-polymer nanostructures and storing them for purposes of characterization and/or use.

The process developed according to the invention has allowed to produce nanometric shell-core structures, smaller than 300 nm, stable and endowed with mucoadhesive properties.

According to another aspect thereof, the invention also relates to a process for the production of chitosan-coated lipid nanostructures loaded with active molecules, to be used for pharmaceutical, nutraceutical, cosmetic, personal hygiene purposes, for food purposes or as dyes.

According to yet another aspect thereof, the invention also relates to pharmaceuticals, nutraceuticals, cosmetics, personal hygiene products, foodstuffs or dyes containing chitosan-coated nanoliposomes or lipid nanostructures obtained by carrying out the process according to the invention.

## Brief description of the figures

The specific features of the invention, as well as the advantages of the same, will be more evident with reference to the accompanying drawings, in which:

**Figure 1** shows an exemplary plant scheme for realizing the process of the present invention;
**Figure 2** shows the images obtained by transmission electron microscopy (TEM) of A) uncoated nanoliposomes, B) nanoliposomes coated with chitosan by the drop-wise method and C) nanoliposomes coated with chitosan by the process of the present invention;
**Figure 3** shows in detail, by means of images obtained by transmission electron microscopy (TEM), the structural differences between polymer-coated nanoliposomes obtained by the drop-wise technique (B) and those obtained by the process of the present invention (C);
**Figure 4** shows, in the form of a histogram, a comparison between the percentage of mucoadhesiveness of uncoated liposomes, of liposomes coated with the process of the present invention and of liposomes coated with the drop-wise technique;
**Figure 5** shows the results of stability tests obtained after incubation of nanoliposomes as such, and of the lipid-polymer nanostructures coated through the simil-microfluidic process according to the invention and with the drop-wise technique.

## EXAMPLES

Some specific embodiments of the production process according to the invention are described below, purely by way of non-limiting examples, along with the results of the experimentation carried out.

## Plant scheme

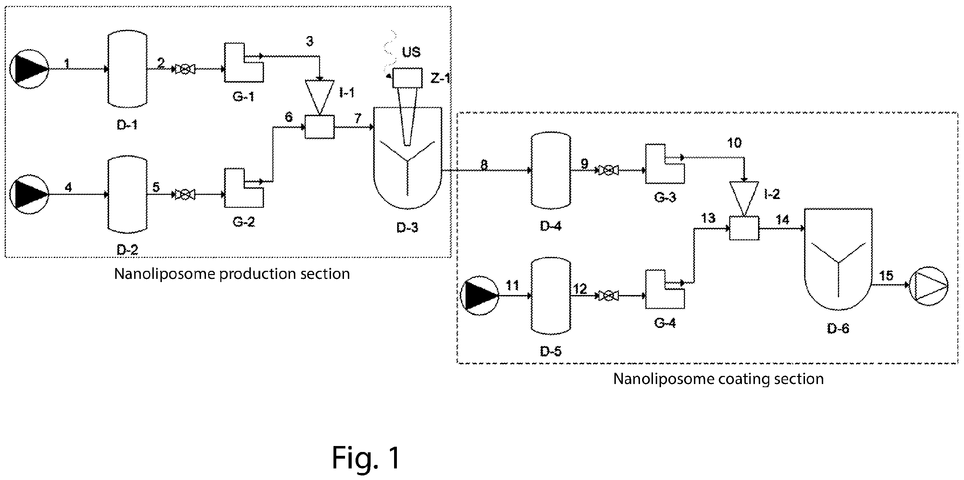

**Figure 1** shows a plant scheme for the realization of the process of the present invention.
The starting liposomal suspension can be produced for example by one of the following techniques, described in the prior art section of the previous unpublished Italian patent application No. 102017000099627:

- production by freeze-thawing (FT), with cooling and heating cycles;
- production by controlled hydration of lipid films or thin-film hydration (TF);
- production by injection of solvent, in particular ethanol, ethanol injection method (EI);
- high pressure homogenization in microfluidizer (MF);
- production by sonication and evaporation of the solvent (reverse-phase evaporation, REV).

According to a preferred solution, however, the starting liposomal suspension is obtained by applying the simil-microfluidic technique described in the previous patent application, and summarized in Figure 1 of the same, which is here reproduced in the first section of the current Figure 1.
In the section of the diagram in Figure 1 related to the nanoliposomes production, an alcoholic solution of the lipid components (possibly containing lipophilic active ingredients) is stored in the tank D-1, from which it is pumped into the supply line (1-2-3) of the alcohol solution by means of the peristaltic pump G-1 to the injector 1-1, which constitutes the area of production of nanoliposomes. The aqueous solution (possibly containing hydrophilic active ingredients) is stored in the tank D-2, from which it is pumped into the supply line (4-5-6) of the aqueous solution by means of the peristaltic pump G-2, also to the injector I-1.

The resulting hydroalcoholic nanoliposomes suspension (7) is then sent to the ultrasound device for homogenization/reduction of the average size, D-3, in which it is subjected to a sonication treatment with working cycles Z-1 (Barba, Bochicchio, Lamberti, & Dalmoro, Ultrasonic energy in liposome production: process modelling and size calculation, 2014, Soft Matter, 10(15), pp. 2574-2581). The homogenized nanoliposomes suspension is recovered through the line (8).

In the section of the diagram in **Figure 1** relating to the coating of the nanoliposomes, the subsequent coating of the nanoliposomes produced is as follows: the liposomal suspension, stored in the D-4 tank, is pumped into the supply line (8-9-10) of the nanoliposomes by means of the peristaltic pump G-3 to the injector I-2 (area of polymer coating of the nanoliposomes). The aqueous chitosan solution, stored in the tank D-5, is pumped into the feed line (11-12-13) of the polymeric solution by the peristaltic pump G-4 to the injector I-2, where the two flows meet. The suspension of the nanoliposomes coated with chitosan is sent to the mixing vessel, D-6, and is then recovered through the line 15.

## Production of shell-core nanostructures: comparison between drop-wise technique and simil-microfluidic technique

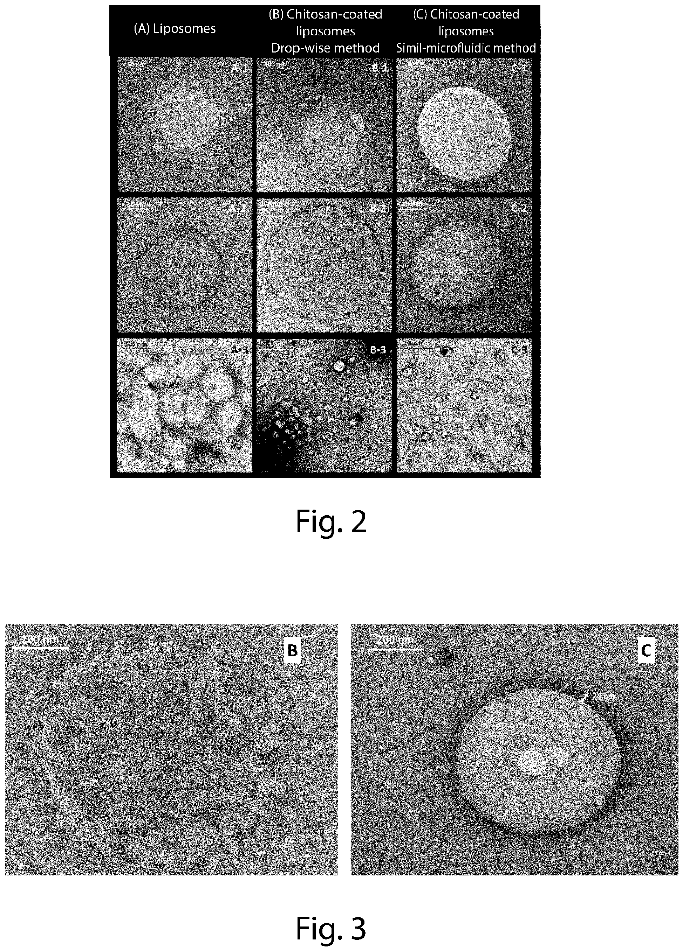

**Figure 2** shows the images obtained by transmission electron microscopy (TEM) of uncoated nanoliposomes, nanoliposomes coated with chitosan by the drop-wise method and nanoliposomes coated with chitosan by the simil-microfluidic method of the present invention. The chitosan layer surrounding the liposomes is clearly visible on the surface of the coated particles obtained through both methods but with obvious differences: if the coating made by the method of the invention provides particles coated with a thick, smooth and homogeneous polymeric surface (C1-C2) those obtained with the drop-wise method show a very thin, rough and irregular coating (B1-B2). These differences, in terms of superficial polymeric distribution and coating thickness, can be appreciated even more in detail in **Figure 3,** which shows, in comparison, in B) nanoliposomes coated with chitosan obtained by drip technique (drop-wise) and in C) chitosan-coated nanoliposomes obtained by the method according the present invention.

## Mucoadhesivity test

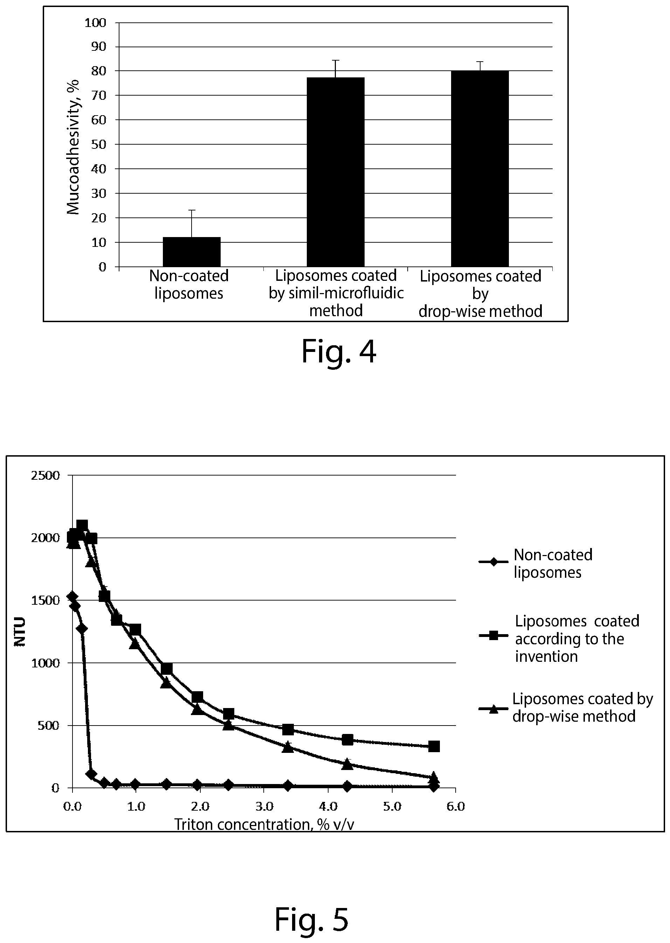

**Figure 4** shows, in the form of a histogram, the mucoadhesive properties of nanoliposomes as such and those coated with chitosan by means of the drop-wise method and the simil-microfluidic method which is the object of the present invention. Mucoadhesivity is expressed as "bin-ding" efficiency of the mucin by the following equation:

$$\text{Mucoadhesivity } \% = (C_0 - C_S)/C_0 * 100$$

where $C_0$ is the initial concentration of mucin present in the liposomes/mucin solution and $C_S$ is the concentration of free mucin in the supernatant after centrifugation of the tested samples.

It can be observed that the mucoadhesive effect is only 10% for the uncoated vesicles compared to about 80% found for the nanoliposomes coated with the two methods (simil-microfluidic technique and drop-wise technique).

## Stability test

The solubilization of liposomes in the presence of detergent may be used as a tool for assessing the structural stability of vesicles and for chitosan-lipid interaction studies.

Figure 5 shows the results of the stability tests obtained after incubation of nanoliposomes as such and polymer-lipid nanostructures (coated with the simil-microfluidic and drop-wise methods) with the Triton X100 detergent. The results of these tests are expressed in nephelometric turbidity units (NTU) as a function of increasing detergent concentrations.

It may be seen that, with the same quantity of Triton X100 added to the samples, there is a clear difference in the solubilization profile between uncoated and chitosan-coated vesicles. While nanoliposomes as they are present a sharp drop in NTU and at a concentration of 0.3% v/v of Triton X100 are already almost completely dissolved (about 93% of the sample is solubilized), at the same concentration of detergent only 0.6% of the sample is solubilized if the coating is carried out by the simil-microfluidic method, and about 7% of the vesicles is dissolved when the coating is carried out by the drop-wise technique. Finally, with the maximum concentration of detergent used (5.7% v/v), the uncoated vesicles and those recovered with the drop-wise method are entirely solubilized while 17% of the nanoliposomes coated with the simil-microfluidic method is still intact.

From the foregoing it arises that the technique object of the invention provides a more uniform coating with respect to what may be obtained with the drop-wise technique, thus conferring greater stability to the coated nanostructures.

The present invention has been described with particular reference to some embodiments thereof but it should be understood that changes and modifications can be made by those skilled in the art without departing from the scope

## Claims

1. Chitosan-coated nanoliposomes or chitosan-coated lipid nanostructures for the administration of active molecules for use as pharmaceutical, nutraceutical or cosmetic agents, obtainable from a process comprising the following operations:

   a) providing, as a starting material, a suspension of nanoliposomes encapsulating one or more active ingredients to be administered, said nanoliposomes being made of phospholipids and cholesterol, preferably phosphatidylcholine and cholesterol, at a total lipid concentration of from 0.1 to 10 mg/mL;
   b) producing an aqueous solution containing chitosan at a chitosan concentration of from 0.005% to 0.015% w/v;
   c) pumping said nanoliposomes suspension and said aqueous solution containing chitosan through two volumetric pumps in a volume flow rate ratio of from 0.8:1 to 1,2:1;
   d) contacting the flows consisting of said nanoliposomes suspension and said aqueous solution containing chitosan by making them pass through a simil-microfluidic device, which effects an inter-diffusion of said nanoliposomes suspension into the stream of said aqueous solution by passage through a duct, for instance a hollow needle, of internal diameter below 1 mm, the duct of said aqueous solution having an internal diameter of 2-5 mm and a length after the injection point of 150-200 mm, thereby obtaining chitosan-coated nanostructures;
   e) collecting the nanostructures obtained in the previous production step.

2. Coated nanoliposomes or lipid nanostructures according to claim 1, wherein said nanoliposomes of operation a) are made of phosphatidylcholine and cholesterol, used in a molar ratio of 2.5:1, and the total lipid concentration is 5 mg/mL.

3. Coated nanoliposomes or lipid nanostructures according to claims 1 or 2, wherein said aqueous solution containing chitosan contains from 0.2% to 1.0% by volume of an organic or inorganic acid.

4. Coated nanoliposomes or lipid nanostructures according to claim 3, wherein said acid is an organic acid selected from the group consisting of: acetic acid, adipic acid, formic acid, lactic acid, malic acid, propionic acid and succinic acid.

5. Coated nanoliposomes or lipid nanostructures according to claim 4, wherein said chitosan-containing aqueous solution contains 0.5% by volume of acetic acid.

6. Coated nanoliposomes or lipid nanostructures according to any one of the preceding claims, wherein said chitosan-containing aqueous solution contains not more than 0.01% w/v of chitosan, and preferably contains 0.01% w/v of chitosan.

7. Coated nanoliposomes or lipid nanostructures according to any one of the preceding claims, wherein said operation c) is carried out by pumping said nanoliposomes suspension and said aqueous solution containing chitosan at the same volume flow rate, said volume flow rate being preferably 25 mL/min.

8. Coated nanoliposomes or lipid nanostructures according to any one of the preceding claims, wherein the ratio of the internal diameter of the duct of said nanoliposomes suspension to the internal diameter of the duct of said aqueous solution is 1:5.

9. A continuous or semi-continuous process for the preparation of nanoliposomes or lipid nanostructures coated with chitosan which comprises the operations a) to e) as described in any one of claims 1-8.

10. Pharmaceutical, nutraceutic or cosmetic products, products for personal care, food products or colorants containing nanoliposomes or lipid nanostructures coated with chitosan obtained by carrying out the process according to claim 9.

## Patentansprüche

1. Chitosan-beschichtete Nanoliposome oder chitosan-beschichtete Lipid-Nanostrukturen zur Verabreichung aktiver Moleküle zur Verwendung als pharmazeutische, nutrazeutische oder kosmetische Mittel, die aus einem Verfahren erhältlich sind, das die folgenden Vorgänge umfasst:

   a) Bereitstellen, als Ausgangsmaterial, einer Suspension aus Nanoliposomen, die einen oder mehrere zu verabreichende Wirkstoffe ummanteln, wobei die Nanoliposome aus Phospholipiden und Cholesterin, vorzugsweise Phosphatidylcholin und Cholesterin mit einer Gesamtlipidkonzentration von 0,1 bis 10 mg/mL hergestellt sind;
   b) Erzeugen einer wässrigen Lösung, die Chitosan mit einer Chitosankonzentration von 0,005% bis 0,015% an Gewicht pro Volumen enthält;
   c) Pumpen der Nanoliposom-Suspension und der Chitosan enthaltenden wässrigen Lösung durch zwei volumetrische Pumpen in einem Volumenstromratenverhältnis von 0,8:1 bis 1,2:1;

d) In-Kontakt-bringen der Strömungen der Nanoliposom-Suspension und der Chitosan enthaltenden wässrigen Lösung, indem man sie durch eine Simil-Mikrofluidvorrichtung laufen lässt, was eine Interdiffusion der Nanoliposom-Suspension in den Strom der wässrigen Lösung durch den Durchlass durch einen Kanal, beispielsweise eine hohle Nadel, mit einem Innendurchmesser von unter 1 mm bewirkt, wobei der Kanal der wässrigen Lösung einen Innendurchmesser von 2-5 mm und eine Länge nach dem Injektionspunkt von 150-200 mm aufweist, wodurch man chitosan-beschichtete Nanostrukturen erhält;

e) Sammeln der im vorstehenden Erzeugungsschritt erhaltenen Nanostrukturen.

2. Beschichtete Nanoliposome oder Lipid-Nanostrukturen nach Anspruch 1, wobei die Nanoliposome aus Vorgang a) aus Phosphatidylcholin und Cholesterin hergestellt sind, die in einem Molverhältnis von 2,5:1 verwendet werden, und die Gesamtlipidkonzentration 5 mg/mL beträgt.

3. Beschichtete Nanoliposome oder Lipid-Nanostrukturen nach Anspruch 1 oder 2, wobei die wässrige Lösung, die Chitosan enthält, von 0,2 Vol.-% bis 1,0 Vol.-% organischer oder anorganischer Säure enthält.

4. Beschichtete Nanoliposome oder Lipid-Nanostrukturen nach Anspruch 3, wobei die Säure eine organische Säure ist, die aus der Gruppe ausgewählt ist, bestehend aus: Essigsäure, Adipinsäure, Ameisensäure, Milchsäure, Apfelsäure, Propionsäure und Bernsteinsäure.

5. Beschichtete Nanoliposome oder Lipid-Nanostrukturen nach Anspruch 4, wobei die Chitosan enthaltende wässrige Lösung 0,5 Vol.-% an Essigsäure enthält.

6. Beschichtete Nanoliposome oder Lipid-Nanostrukturen nach einem der vorstehenden Ansprüche, wobei die Chitosan enthaltende wässrige Lösung nicht mehr als 0,01% an Gewicht pro Volumen an Chitosan enthält, und vorzugsweise 0,01% an Gewicht pro Volumen an Chitosan enthält.

7. Beschichtete Nanoliposome oder Lipid-Nanostrukturen nach einem der vorstehenden Ansprüche, wobei der Vorgang c) durch Pumpen der Nanoliposom-Suspension und der Chitosan enthaltenden wässrigen Lösung mit derselben Volumenstromrate ausgeführt wird, wobei die Volumenstromrate vorzugsweise 25 mL/min. beträgt.

8. Beschichtete Nanoliposome oder Lipid-Nanostrukturen nach einem der vorstehenden Ansprüche, wobei das Verhältnis des Innendurchmessers des Kanals der Nanoliposom-Suspension zum Innendurchmesser des Kanals der wässrigen Lösung 1:5 beträgt.

9. Kontinuierliches oder halbkontinuierliches Verfahren zur Herstellung von chitosanbeschichteten Nanoliposomen oder Lipid-Nanostrukturen, das die Vorgänge a) bis e) nach einem der Ansprüche 1-8 umfasst.

10. Pharmazeutische, nutrazeutische oder kosmetische Mittel, Mittel zur Körperpflege, Lebensmittelprodukte oder Farbstoffe, die chitosan-beschichtete Nanoliposome oder Lipid-Nanostrukturen enthalten, die durch Ausführen des Verfahrens nach Anspruch 9 erhalten werden.

**Revendications**

1. Nanoliposomes enrobés de chitosane ou nanostructures lipidiques enrobées de chitosane pour l'administration de molécules actives destinées à être utilisées en tant qu'agents pharmaceutiques, nutraceutiques ou cosmétiques, pouvant être obtenus à partir d'un processus comprenant les opérations suivantes :

a) la fourniture, en tant qu'une matière de départ, d'une suspension de nanoliposomes encapsulant un ou plusieurs ingrédients actifs à administrer, lesdits nanoliposomes étant constitués de phospholipides et de cholestérol, de préférence de phosphatidylcholine et de cholestérol, à une concentration lipidique totale de 0,1 à 10 mg/ml ;

b) la production d'une solution aqueuse contenant du chitosane à une concentration de chitosane de 0,005 % à 0,015 % en poids/volume ;

c) le pompage de ladite suspension de nanoliposomes et de ladite solution aqueuse contenant du chitosane à travers deux pompes volumétriques à un rapport de débit volumétrique de 0,8:1 à 1,2:1 ;

d) la mise en contact des écoulements se composant de ladite suspension de nanoliposomes et de ladite solution aqueuse contenant du chitosane en les faisant passer à travers un dispositif simil-microfluidique, qui effectue une interdiffusion de ladite suspension de nanoliposomes dans le flux de ladite solution aqueuse par un passage à travers un conduit, par exemple une aiguille creuse, de diamètre interne inférieur à 1 mm, le conduit de ladite solution aqueuse ayant un diamètre interne de 2-5 mm et une longueur après le point d'injection de 150-200 mm, en obtenant de ce fait des nanostructures enrobées de chitosane ;

e) la collecte des nanostructures obtenues à l'étape de production précédente.

2. Nanoliposomes ou nanostructures lipidiques enrobés selon la revendication 1, dans lesquels lesdits nanoliposomes de l'opération a) sont constitués de phosphatidylcholine et de cholestérol, utilisés dans un rapport molaire de 2,5:1, et la concentration lipidique totale est 5 mg/ml.

3. Nanoliposomes ou nanostructures lipidiques enrobés selon les revendications 1 ou 2, dans lesquels ladite solution aqueuse contenant du chitosane contient de 0,2 % à 1,0 % en volume d'un acide organique ou inorganique.

4. Nanoliposomes ou nanostructures lipidiques enrobés selon la revendication 3, dans lesquels ledit acide est un acide organique sélectionné dans le groupe se composant de : acide acétique, acide adipique, acide formique, acide lactique, acide malique, acide propionique et acide succinique.

5. Nanoliposomes ou nanostructures lipidiques enrobés selon la revendication 4, dans lesquels ladite solution aqueuse contenant du chitosane contient 0,5 % en volume d'acide acétique.

6. Nanoliposomes ou nanostructures lipidiques enrobés selon l'une quelconque des revendications précédentes, dans lesquels ladite solution aqueuse contenant du chitosane ne contient pas plus de 0,01 % poids/volume de chitosane, et de préférence contient 0,01 % poids/volume de chitosane.

7. Nanoliposomes ou nanostructures lipidiques enrobés selon l'une quelconque des revendications précédentes, dans lesquels ladite opération c) est réalisée par le pompage de ladite suspension de nanoliposomes et de ladite solution aqueuse contenant du chitosan au même débit volumétrique, ledit débit volumétrique étant de préférence 25 ml/min.

8. Nanoliposomes ou nanostructures lipidiques enrobés selon l'une quelconque des revendications précédentes, dans lesquels le rapport du diamètre interne du conduit de ladite suspension de nanoliposomes sur le diamètre interne du conduit de ladite solution aqueuse est 1:5.

9. Processus continu ou semi-continu pour la préparation de nanoliposomes ou de nanostructures lipidiques enrobés de chitosane comprenant les opérations a) à e) selon l'une quelconque des revendications 1 à 8.

10. Produits pharmaceutiques, nutraceutiques ou cosmétiques, produits d'hygiène personnelle, produits alimentaires ou colorants contenant des nanoliposomes ou nanostructures lipidiques enrobés de chitosane obtenus par la réalisation du processus selon la revendication 9.

Nanoliposome coating section

Nanoliposome production section

Fig. 1

Fig. 2

Fig. 3

Fig. 4

Fig. 5

## REFERENCES CITED IN THE DESCRIPTION

### Patent documents cited in the description

- US 8658204 B
- WO 2006062506 A
- US 20130337047 A1

- US 20140017298 A1
- IT 102017000099627

### Non-patent literature cited in the description

- **KARN, P.R. et al.** Mucoadhesive liposomal delivery systems: the choice of coating material. *Drug development and industrial pharmacy,* 2011, vol. 37 (4), 482-488
- **WU, Z.-H. et al.** Hypoglycemic efficacy of chitosan-coated insulin liposomes after oral administration in mice. *Acta Pharmacologica Sinica,* 2004, vol. 25 (7), 966-972
- **GUO, J.-X. et al.** Chitosan-coated liposomes: characterization and interaction with leuprolide. *International Journal of pharmaceutics,* 2003, vol. 260 (2), 167-173
- **BANG et al.** *Journal of Microencapsulation,* vol. 28 (7), 595-604
- **GONÇALVES, M. C. et al.** Chitosan coated liposomes as an innovative nanocarrier for drugs. *Journal of biomedical nanotechnology,* 2012, vol. 8 (2), 240-250
- **OTAKE, K. et al.** One-step preparation of chitosan-coated cationic liposomes by an improved supercritical reverse-phase evaporation method. *Langmuir,* 2006, vol. 22 (9), 4054-4059
- **MEURE, L. A. et al.** Conventional and dense gas techniques for the production of liposomes: a review. *Aaps Pharmscitech,* 2008, vol. 9 (3), 798-809
- **PISTONE, S. et al.** Polysaccharide-coated liposomal formulations for dental targeting. *International Journal of Pharmaceutics,* 2017, vol. 516 (1), 106-115

- **GE, L. ; JI, J.** Fabrication and stability study of multilayer liposome. *Asian Journal of Chemistry,* 2010, vol. 22 (1), 66
- *CHEMICAL ABSTRACTS,* 53-86-1
- *CHEMICAL ABSTRACTS,* 863-61-6
- *CHEMICAL ABSTRACTS,* 10191-41-0
- *CHEMICAL ABSTRACTS,* 58-27-5
- *CHEMICAL ABSTRACTS,* 67-97-0
- *CHEMICAL ABSTRACTS,* 458-37-7
- **DEYGEN, I. M. et al.** Novel Prodrug of Doxorubicin Modified by Stearoylspermine Encapsulated into PEG-Chitosan-Stabilized Liposomes. *Langmuir,* 2016, vol. 32, 10861-10869
- **WERLE, M. et al.** Chitosan-aprotinin coated liposomes for oral peptide delivery: development, characterization and in vivo evaluation. *International Journal of Pharmaceutics,* 2009, vol. 370 (1-2), 26-32
- **CHANNARONG, S. et al.** Development and evaluation of chitosan-coated liposomes for oral DNA vaccine: the improvement of Peyer's patch targeting using a polyplex-loaded liposomes. *AAPS Pharmscitech,* 2011, vol. 12 (1), 192-200
- **BARBA ; BOCHICCHIO ; LAMBERTI ; DALMORO.** Ultrasonic energy in liposome production: process modelling and size calculation. *Soft Matter,* 2014, vol. 10 (15), 2574-2581